# EUROPÄISCHE PATENTSCHRIFT

(11) **EP 0 576 652 B1**
(45) Veröffentlichungstag und Bekanntmachung des Hinweises auf die Patenterteilung: **06.03.1996**
(21) Anmeldenummer: 93901684.6
(22) Anmeldetag: 13.01.1993
(51) Int. Cl.: A61B 17/70

(54) **IMPLANTAT ZUR AUSRICHTUNG UND FIXIERUNG ZWEIER KNOCHEN ODER KNOCHENTEILE ZUEINANDER, INSBESONDERE SPONDYLODESE-IMPLANTAT**
IMPLANT FOR SETTING AND FIXING TWO BONES OR PARTS THEREOF TOGETHER, ESPECIALLY A SPONDYLODESIS IMPLANT
IMPLANT POUR LA REDUCTION ET LA FIXATION DE DEUX OS OU PARTIES D'OS ENSEMBLE, EN PARTICULIER IMPLANT DE SPONDYLODESE

(30) Priorität: 16.01.1992 DE 4200905
(43) Veröffentlichungstag der Anmeldung: 05.01.1994
(73) Patentinhaber: ULRICH, Heinrich, D-89077 Ulm/Donau (DE)
(72) Erfinder: ULRICH, Heinrich, D-89077 Ulm/Donau (DE)
(74) Vertreter: Fay, Hermann, Dipl.-Phys. Dr.
(86) Internationale Anmeldenummer: DE9300033
(87) Internationale Veröffentlichungsnummer: WO9313723

(56) Entgegenhaltungen:
- EP-A- 0 348 581
- EP-A- 0 408 489

## Beschreibung

Die Erfindung betrifft ein Implantat zur Ausrichtung und Fixierung zweier Knochen oder Knochenteile zueinander, wie Spondylodese-Implantat zur Korrektur und Fixierung der gegenseitigen Wirbelstellung, mit mindestens zwei relativ zueinander geführten und in Führungsrichtung bezüglich ihres gegenseitigen Abstandes einstellbaren Anschlußstücken, und mit in die Knochen oder Knochenteile, wie Wirbel, einschraubbaren Repositionsschrauben, für die zur Befestigung der Anschlußstücke an den Knochen oder Knochenteilen, wie Wirbeln, Aufnahmen in den Anschlußstücken vorgesehen sind, wobei die Repositionsschrauben in ihrer jeweiligen Aufnahme in Schraubenlängsrichtung verschiebbar und quer dazu verschwenkbar geführt und die Aufnahmen mit einer durch ein Klemmglied betätigbaren Klemmeinrichtung ausgestattet sind, die im Klemmzustand die Repositionsschraube sowohl gegen Verschieben als auch verschwenken in der Aufnahme feststellt, und wobei ein koaxialer Kegelsitz vorgesehen und im Anschlußstück eine Lageröffnung für einen Gelenkkörper und in diesem ein Führungskanal für die Repositionsschraube ausgebildet ist.

Bei aus EP-A-348 581 bekannten Implantaten dieser Art befindet sich der koaxiale Kegelsitz unmittelbar im Anschlußstück und bildet dessen Lageröffnung. In dem koaxialen Kegelsitz befindet sich der als Spannzange für die Repositionsschraube ausgebildete Gelenkkörper, der durch das Klemmglied in den koaxialen Kegelsitz hineingezogen wird. Dazu ist das Klemmglied als eine Mutter ausgebildet, die auf einem Außengewinde an einem aus dem Kegelsitz vortehenden Schaft des Gelenkkörpers geführt und gegen das Anschlußstück abgestützt ist. Um die Repositionsschraube kippen und auch in einer Stellung fixieren zu können, in der sie mit der Achse des Kegelsitzes einen Winkel bildet, weist der durch das Hineinziehen in den Kegelsitz zusammendrückbare Abschnitt des Gelenkkörpers eine kugelförmige Außenfläche auf. Damit beim Verspannen des Gelenkkörpers, also beim Anziehen der Mutter, die gewählte Stellung beibehalten wird, ist zwischen der Mutter und dem Ende des Kegelsitzes eine Unterlagscheibe angeordnet, die einen mit einer Zahnung versehenen kreissegmentartigen Ausschnitt aufweist, wobei die Zahnung mit einer dazu passenden Zahnung am Ende des Kegelsitzes kämmt. Um im übrigen beim Anziehen der Mutter ein Mitdrehen des Gelenkkörpers zu verhindern, ist das Außengewinde am Gelenkkörper mit mindestens einer Abflachung versehen, während der Kegelsitz an seinem engeren Ende eine dazu passende Verengung aufweist, in der der Gelenkkörper gegen Verdrehen gesichert ist.

Der Erfindung liegt die Aufgabe zugrunde, ein Implantat der eingangs genannten Art in seinem Aufbau zu vereinfachen und so auszubilden, daß die Repositionsschrauben weitgehend unabhängig von ihrer Einschraubtiefe und Ausrichtung am Knochen oder Wirbel in einfach und schnell handhabbarer Weise in ihren Aufnahmen an den Anschlußstücken zuverlässig befestigt werden können.

Diese Aufgabe wird bei einem Implantat der eingangs genannten Art erfindungsgemäß dadurch gelöst, daß die Repositionsschraube beidseits des Gelenkkörpers durch in der Wandung des Anschlußstücks vorgesehene Fenster greift, daß der Gelenkkörper quer zur Achse des Führungskanals zweigeteilt und in einem der beiden Teile der mit dem Führungskanal koaxiale Kegelsitz ausgebildet ist, in dem sich ein die Repositionsschraube umschließender, längs einer Mantellinie geschlitzter Klemmring befindet, und daß das Klemmglied im Klemmzustand die beiden Gelenkkörperteile in der Lageröffnung gegen deren Lagerfläche und den Klemmring Kegelsitz gegen die Repositionsschraube verspannt.

Durch die Erfindung wird erreicht, daß die Aufnahme die Positionierung der Repositionsschraube am Knochen bezüglich ihrer Einschraubtiefe und Ausrichtung weitgehend unbeeinflußt läßt, da bei noch offener Klemmeinrichtung die positionsschraube in der Aufnahme sowohl verschiebbar als auch verschwenkbar ist. Jedoch werden diese Bewegungsmöglichkeiten auf konstruktiv besonders einfache Weise gemeinsam blockiert, also die Repositionsschraube und das Anschlußstück starr miteinander verbunden, sobald das Klemmglied entsprechend betätigt und dadurch die Klemmeinrichtung wirksam wird.

Die gemeinsame Verklemmung sowohl der Gelenkkörperteile einerseits und des Klemmrings andererseits kann in besonders vorteilhafter Weise dadurch verwirklicht werden, daß der Kegelsitz mit dem erweiterten Ende im Spalt zwischen beiden Gelenkkörperteilen mündet und in dem dieser Mündung gegenüber liegenden Gelenkkörperteil eine mit dem Führungskanal koaxiale Gewindebohrung vorgesehen ist, in der als Klemmglied eine einen Abschnitt des Führungskanales bildende Gewindehülse geführt ist, die mit dem innerhalb des Gelenkkörpers liegenden Hülsenende gegen den im Kegelsitz befindlichen Klemmring stößt und am anderen Hülsenende durch das Fenster nach außen vorsteht und zur Betätigung zugänglich ist. Dabei empfiehlt es sich, daß der Klemmring eine dem Kegelsitz angepaßte äußere Kegelfläche und der der Gewindehülse zugewandte Rand des Klemmrings einen mindestens so großen Außendurchmesser wie die Gewindehülse besitzt, wodurch erreicht wird, daß sich die Gewindehülse beim Anziehen immer nur gegen den Klemmring, nie aber direkt gegen den mit dem Kegelsitz versehenen Gelenkkörperteil abstützen kann.

In der Regel genügt es, die Verschwenkbarkeit der Repositionsschraube gegenüber dem Anschlußstück nur um eine einzige Achse zu ermöglichen. In diesem Fall empfiehlt es sich, die Anordnung im einzelnen so zu treffen, daß die Lageröffnung und der Gelenkkörper zylindrisch mit zur Achse des Führungskanales senkrechter Zylinderachse ausgebildet sind, daß in beidseits des Führungskanales an der äußeren Umfangsfläche des Gelenkkörpers koaxial mit der Zylinderachse ausgebildete Ringnuten längs einer Mantellinie geschlitzte Federringe eingelegt sind, die außenseitig mit einer Querrillierung versehen sind und mit den Rillenstegen radial geringfügig über die zylindrische Umfangsfläche des Gelenkkörpers vorstehen. Bei der Verspannung der Gelenkkörperteile können sich dann die Rillenstege einerseits in die zylindrische Umfangsfläche der Lageröffnung, andererseits in die Wandung der Ringnuten eindrücken und so eine besonders starre und zuverlässige Sicherung des Gelenkkörpers gegen Verdrehen ergeben. Die Querrillierung kann in einfachster Ausführungsform von einem auf den Federring aufgebrachten Gewinde gebildet sein. Die Verschwenkbarkeit des Gelenkkörpers um nur eine einzige Achse empfiehlt sich besonders bei Implantaten, bei denen die Führung des Anschlußstückes an einer Spindel erfolgt, um deren Achse das Anschlußstück verdrehbar ist und sich daher einer seiner Verdrehbarkeit entsprechenden Schrägstellung der Repositionsschraube anpassen kann. In diesem Fall steht daher vorzugsweise die Zylinderachse des Gelenkkörpers senkrecht zur Spindelachse.

Im folgenden wird die Erfindung an in der Zeichnung dargestellten Ausführungsbeispielen näher erläutert; es zeigen:
- Fig. 1: eine Seitenansicht eines Spondylodese-Implantates nach der Erfindung,
- Fig. 2: eine Draufsicht auf das Implantat nach Fig. 1,
- Fig. 3: eine andere Ausführungsform des Implantates in einer der Fig. 1 entsprechenden Darstellung,
- Fig. 4: das Implantat nach Fig. 3 in einer Draufsicht,
- Fig. 5: eine Stirnansicht der Implantate nach den Fig. 1 bis 4 in Richtung der Pfeile V,

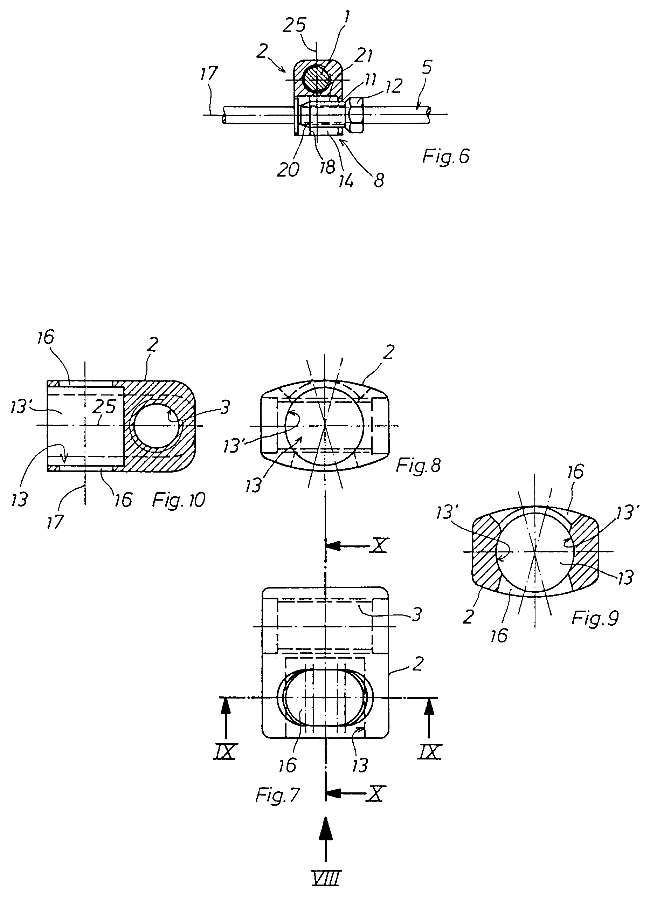

- Fig. 6: einen Schnitt in Richtung IV - IV durch die Implantate nach den Fig. 1 bis 5,
- Fig. 7: eine Draufsicht nur auf das Anschlußstück des Gegenstandes der Fig. 6,
- Fig. 8: eine Seitenansicht des Anschlußstückes der Fig. 7 in Richtung des dort eingetragenen Pfeiles VIII,
- Fig. 9: den Schnitt IX - IX in Fig. 7,
- Fig. 10: den Schnitt X - X in Fig. 7,

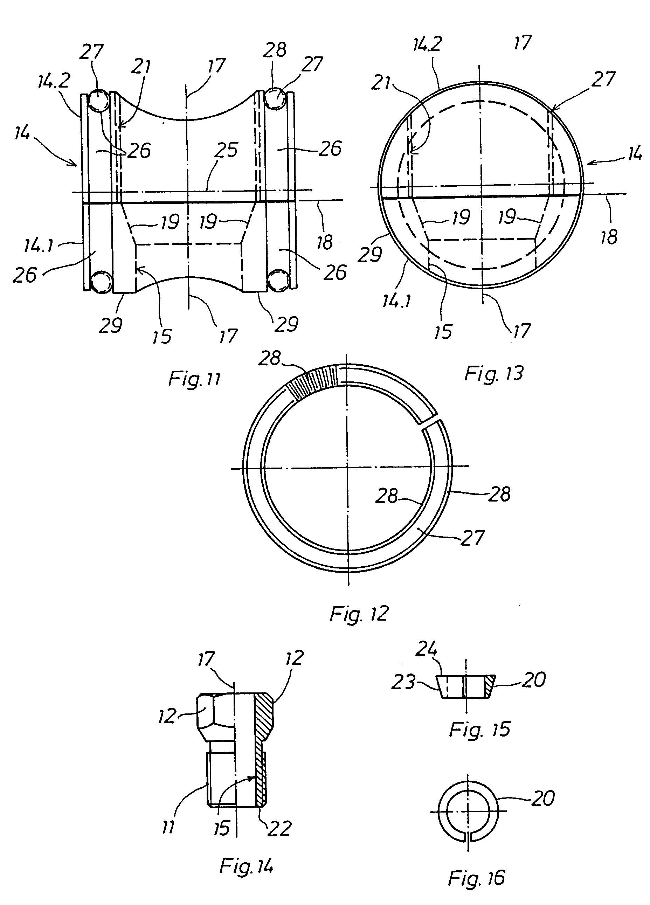

- Fig. 11: den Gelenkkörper des Gegenstandes der Fig. 6 zusammen mit den Federringen in einer Seitenansicht,
- Fig. 12: die Federringe in Fig. 11 in axialer Ansicht,
- Fig. 13: den Gelenkkörper der Fig. 11 in axialer Ansicht,
- Fig. 14: die Gewindehülse,
- Fig. 15: den Klemmring jeweils des Gegenstandes der Fig. 6, teils in Seitenansicht, teils im Schnitt, und
- Fig. 16: eine Axialansicht des Klemmrings nach Fig. 15.

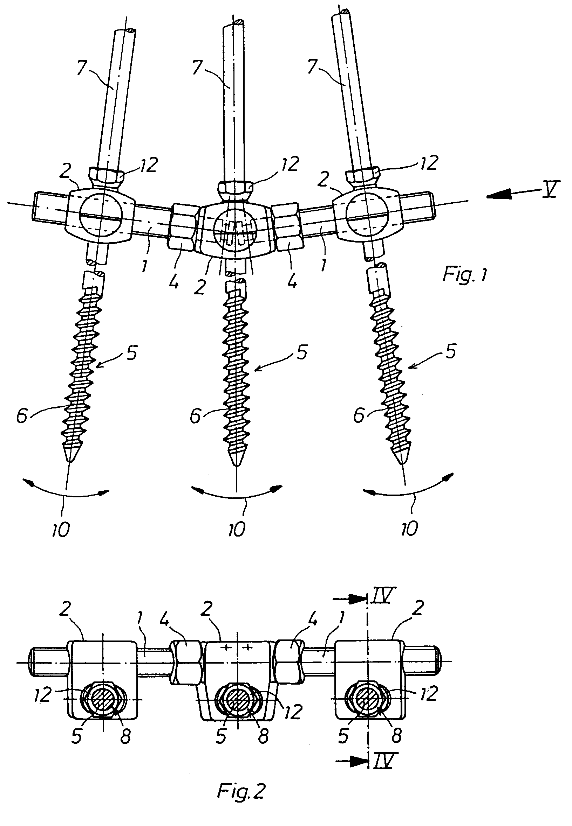

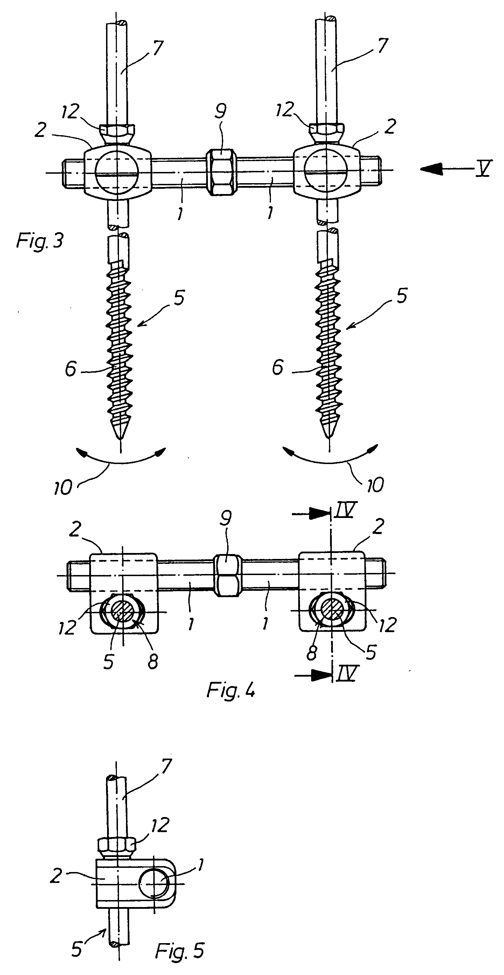

Die Fig. 1 bis 5 zeigen Spondylodese-Implantate, bei denen an Gewindespindeln 1 Anschlußstücke 2 geführt sind, die durch Verdrehen der Gewindespindel 1 in ihrem gegenseitigen Abstand voneinander verstellbar sind. Im Ausführungsbeispiel nach den Fig. 1 und 2 sind insgesamt drei Anschlußstücke 2 vorgesehen, wobei das mittlere Anschlußstück dazu dient, die beiden seitlichen Gewindespindeln 1 drehbar und axial unverschiebbar zu lagern, was im einzelnen in der Zeichnung nicht dargestellt ist. Die beiden Gewindespindeln 1 laufen in Muttergewinden 3 der seitlichen Anschlußstücke, so daß durch Verdrehen der Gewindespindeln 1, die dafür zum Ansatz eines geeigneten Schlüssels mit einem Sechskant 4 ausgestattet sind, die beiden seitlichen Anschlußstücke 2 bezüglich ihres Abstandes vom mittleren Anschlußstück 2 unabhängig voneinander eingestellt werden können. Zur Verbindung der Anschlußstücke 2 mit jeweils einem in der Zeichnung nicht dargestellten Wirbel dienen Knochenschrauben 5, die mit einem Gewindeabschnitt 6 in den Wirbel einschraubbar sind und mit einem glatten, gewindelosen Schaftabschnitt 7 in einer allgemein mit 8 bezeichneten Aufnahme der Anschlußstücke 2 gehalten sind. - Das Implantat nach den Fig. 3 bis 5 unterscheidet sich von dem Implantat nach den Fig. 1 und 2 im wesentlichen nur darin, daß lediglich zwei Anschlußstücke 2 vorhanden sind und ihre beiden Gewindespindeln 1 einstückig miteinander ausgebildet sind. Zwischen beiden Anschlußstücken 2 sind die Gewindespindeln zur gemeinsamen Betätigung durch einen Schlüssel wiederum mit einem Sechskant 9 ausgestattet. Beidseits dieses Sechskants besitzen die Gewindespindeln 1 entgegengesetzten Gewindesinn, was entsprechend auch für die Muttergewinde der beiden Anschlußstücke 2 gilt. Wird daher die einstückige Gewindespindel 1 verdreht, werden je nach Drehrichtung die beiden Anschlußstücke 2 aufeinander zu- oder voneinander fortbewegt.

Die Aufnahmen 8 für die Repositionsschrauben 5 in den Anschlußstücken 2 sind bei beiden Implantaten gleich ausgebildet. Dabei sind die Repositionsschrauben 5 in ihrer jeweiligen Aufnahme 8 in Schraubenlängsrichtung mit dem gewindelosen glatten Schaftteil 7 verschiebbar geführt und quer dazu in Richtung der Pfeile 10 auch verschwenkbar. Im übrigen sind die Aufnahmen 8 mit einer durch ein Klemmglied betätigbaren Klemmeinrichtung ausgestattet, die im Klemmzustand die Repositionsschraube 5 sowohl gegen Verschieben als auch Verschwenken in der Aufnahme 8 feststellt. Das Klemmglied ist eine Gewindehülse 11, die zur Betätigung mittels eines Schlüssels wiederum mit einem Sechskant 12 ausgestattet ist.

Im einzelnen ist im Anschlußstück 2 eine Lageröffnung 13 für einen Gelenkkörper 14 und im Gelenkkörper 14 ein Führungskanal 15 für die Repositionsschraube 5 ausgebildet. Die Repositonsschraube 5 steht beidseits des Gelenkkörpers 14 durch in der Wandung des Anschlußstücks 2 vorgesehene Fenster 16 aus dem Anschlußstück 2 vor. Der Gelenkkörper 14 ist quer zur Achse 17 des Führungskanals 15 in der Ebene 18 zweigeteilt. In einem 14.1 der beiden Teile 14.1, 14.2 ist ein mit dem Führungskanal 15 koaxialer Kegelsitz 19 ausgebildet, in dem sich ein die Repositionsschraube 5 umschließender, längs einer Mantellinie geschlitzter Klemmring 20 befindet. Das Klemmglied, also die Gewindehülse 11 verspannt im Klemmzustand einerseits die beiden Gelenkkörperteile 14.1, 14.2 in der Lageröffnung 13 gegen deren Lagerfläche 13' und andererseits den Klemmring 20 im Kegelsitz 19 gegen die Repositionsschraube 5. Das wird in einfacher Weise dadurch erreicht, daß der Kegelsitz 19 mit dem erweiterten Ende im Spalt 18 zwischen beiden Gelenkkörperteilen 14.1, 14.2 mündet und in dem dieser Mündung gegenüber liegenden Gelenkkörperteil 14.2 eine mit dem Führungskanal 15 koaxiale Gewindebohrung 21 vorgesehen ist, in der die einen Abschnitt des Führungskanals 15 bildende Gewindehülse 11 geführt ist. Die Gewindehülse 11 stößt mit dem innerhalb des Gelenkkörpers 14 liegenden Hülsenende 22 gegen den im Kegelsitz 19 befindlichen Klemmring 20 und steht am anderen Hülsenende mit dem dort befindlichen Sechskant 12 durch das Fenster 16 nach außen vor, so daß der Sechskant 12 zur Betätigung der Gewindehülse 11 zugänglich ist. Der Klemmring 20 besitzt eine dem Kegelsitz 19 angepaßte äußere Kegelfläche 23. Der der Gewindehülse 11 zugewandte Rand 24 des Klemmrings 20 besitzt einen mindestens so großen Außendurchmesser wie das Ende 22 die Gewindehülse 11. Im Ergebnis stützt sich die Gewindehülse 11 immer auf dem ihr zugewandten Rand 24 des Klemmrings 20 ab. Wird also die Gewindehülse 11 angezogen, wird der Klemmring 20 im Kegelsitz 19 gegen den gewindelosen Schaftteil 7 der Repositionsschraube 5 verspannt; zugleich werden durch die sich an dem Klemmring 20 abstützende Gewindehülse 11 die beiden Gelenkkörperteile 14.1, 14.2 auseinander gedrückt und dadurch in der Lageröffnung 13 verklemmt.

Der Gelenkkörper 14 und die Lageröffnung 13 sind in den Ausführungsbeispielen zylindrisch mit zur Achse 17 des Führungskanales 15 senkrechter Zylinderachse 25 ausgebildet. Die Verschwenkbarkeit des Gelenkkörpers 14 um diese nur eine einzige Achse 25 genügt im Fall der Ausführungsbeispiele, da die Anschlußstücke 2 selbst um die Achse der sie führenden Gewindespindeln 1 verdrehbar sind und sich daher einer Schrägstellung der Repositionsschrauben 5 im Sinne dieser Verdrehmöglichkeit auf der Gewindespindel 1 anpassen können. Bei bezüglich der Führung der Anschlußstücke 2 anders ausgebildeten Implantaten ist es aber selbstverständlich grundsätzlich möglich, Lageröffnungen und entsprechende Gelenkkörper, beispielsweise Kugelgelenkkörper vorzusehen, die auch eine Verdrehung um zwei zueinander senkrechte Achsen ermöglichen. Der Aufbau einer solchen Aufnahme 8 ist dann allerdings aufwendiger. - Im vorliegenden Fall nur zylindrischer Gelenkkörper 14 besteht eine besonders einfache Möglichkeit, eine sehr wirksame Verklemmung des Gelenkkörpers 14 in der Lageröffnung 13 und damit eine sichere Fixierung der Repositionsschrauben 5 gegen Verschwenken zu erreichen. Dazu sind beidseits des Führungskanals 15 an der äußeren Umfangsfläche des Gelenkkörpers 14 koaxial mit der Zylinderachse 25 verlaufende Ringnuten 26 ausgebildet, in die längs einer Mantellinie geschlitzte Federringe 27 eingelegt sind, die außenseitig mit einer Querrillierung 28 versehen sind und mit den Rillenstegen radial geringfügig über die zylindrische Umfangsfläche 29 des Gelenkkörpers 14 vorstehen. Diese Querrillierung 28 ist dabei in einfacher Weise durch ein auf den Federring 27 aufgebrachtes Gewinde gebildet. Werden daher bei Betätigung der Gewindehülse 11 die beiden Gelenkkörperteile 14.1, 14.2 auseinander gedrückt, erfolgt deren Verspannung gegen die Lagerfläche 13' der Lageröffnung 13 über die Federringe 27, wobei sich deren Rillenstege einerseits in die Lagerfläche 13' der Lageröffnung 13, andererseits in die Wandung der Ringnuten 26 eingraben.

## Patentansprüche

1. Implantat zur Ausrichtung und Fixierung zweier Knochen oder Knochenteile zueinander, wie Spondylodese-Implantat zur Korrektur und Fixierung der gegenseitigen Wirbelstellung, mit mindestens zwei relativ zueinander geführten und in Führungsrichtung bezüglich ihres gegenseitigen Abstandes einstellbaren Anschlußstücken (2), und mit in die Knochen oder Knochenteile, wie Wirbel, einschraubbaren Repositionsschrauben (5), für die zur Befestigung der Anschlußstücke (2) an den Knochen oder Knochenteilen, wie Wirbeln, Aufnahmen (8) in den Anschlußstücken (2) vorgesehen sind, wobei die Repositionsschrauben (5) in ihrer jeweiligen Aufnahme (8) in Schraubenlängsrichtung verschiebbar und quer dazu verschwenkbar geführt und die Aufnahmen (8) mit einer durch ein Klemmglied betätigbaren Klemmeinrichtung ausgestattet sind, die im Klemmzustand die Repositionsschraube (5) sowohl gegen Verschieben als auch Verschwenken in der Aufnahme (8) feststellt, und wobei ein koaxialer Kegelsitz (19) vorgesehen und im Anschlußstück (2) eine Lageröffnung (13) für einen Gelenkkörper (14) und in diesem ein Führungskanal (15) für die Repositionsschraube (5) ausgebildet ist, dadurch gekennzeichnet, daß die Repositionsschraube (5) beidseits des Gelenkkörpers (14) durch in der Wandung des Anschlußstücks (2) vorgesehene Fenster (16) greift, daß der Gelenkkörper (14) quer zur Achse (17) des Führungskanals (15) zweigeteilt und in einem (14.1) der beiden Teile der mit dem Führungskanal (15) koaxiale Kegelsitz (19) ausgebildet ist, in dem sich ein die Repositionsschraube (5) umschließender, längs einer Mantellinie geschlitzter Klemmring (20) befindet, und daß das Klemmglied im Klemmzustand die beiden Gelenkkörperteile (14.1, 14.2) in der Lageröffnung (13) gegen deren Lagerfläche (13') und den Klemmring (20) im Kegelsitz (19) gegen die Repositionsschraube (5) verspannt.

2. Implantat nach Anspruch 1, dadurch gekennzeichnet, daß der Kegelsitz (19) mit dem erweiterten Ende im Spalt (18) zwischen beiden Gelenkkörperteilen (14.1, 14.2) mündet und in dem dieser Mündung gegenüber liegenden Gelenkkörperteil (14.2) eine mit dem Führungskanal (15) koaxiale Gewindebohrung (21) vorgesehen ist, in der als Klemmglied eine einen Abschnitt des Führungskanals (15) bildende Gewindehülse (11) geführt ist, die mit dem innerhalb des Gelenkkörpers (14) liegenden Hülsendende (22) gegen den im Kegelsitz (19) befindlichen Klemmring (20) stößt und am anderen Hülsenende durch das Fenster (16) nach außen vorsteht und zur Betätigung zugänglich ist.

3. Implantat nach Anspruch 1 oder 2, dadurch gekennzeichnet, daß der Klemmring (20) eine dem Kegelsitz (19) angepaßte äußere Kegelfläche (23) und der der Gewindehülse (11) zugewandte Rand (24) des Klemmrings einen mindestens so großen Außendurchmesser wie die Gewindehülse (11) besitzt.

4. Implantat nach einem der Ansprüche 1 bis 3, dadurch gekennzeichnet, daß die Lageröffnung (13) und der Gelenkkörper (14) zylindrisch mit zur Achse (17) des Führungskanals (15) senkrechter Zylinderachse (25) ausgebildet sind, daß in beidseits des Führungskanals (15) an der äußeren Umfangsfläche des Gelenkkörpers (14) koaxial mit der Zylinderachse (25) ausgebildete Ringnuten (26) längs einer Mantellinie geschlitzte Federringe (27) eingelegt sind, die außenseitig mit einer Querrillierung (28) versehen sind und mit den Rillenstegen radial geringfügig über die zylindrische Umfangsfläche (29) des Gelenkkörpers (14) vorstehen.

5. Implantat nach Anspruch 4, dadurch gekennzeichnet, daß die Querrillierung (28) von einem auf den Federring (27) aufgebrachten Gewinde gebildet ist.

6. Implantat nach Anspruch 4 oder 5, dadurch gekennzeichnet, daß bei Führung des Anschlußstückes (2) an einer Spindel (1) des Implantates die Zylinderachse (25) des Gelenkkörpers (14) senkrecht zur Spindelachse steht.

## Claims

1. An implant for orienting and fixing two bones or bone portions relative to each other, such as spondylodesis implant for correcting and fixing the mutual position of vertebrae, comprising at least two connecting portions (2) which are guided relative to each other and which are adjustable in the guidance direction in respect of their mutual spacing, and repositioning screws (5) which can be screwed into the bones or bone portions such as vertebrae and for which receiving means (8) are provided in the connecting portions (2) for fixing the connecting positions (2) to the bones or bone portions such as vertebrae, wherein the repositioning screws (5) are guided in their respective receiving bans (8) slidably in the longitudinal direction of the screw and pivotably transversely with respect thereto and the receiving means (8) are provided with a clamping device which can be actuated by a clamping member and which in the clamping condition fixes the repositioning screw (5) to prevent both sliding movement and also pivotal movement in the receiving means (8), and wherein there is provided a coaxial tapering seat (19) and a mounting opening (13) for a joint body (14) is formed in the connecting portion (2) and a guide passage (15) for the repositioning screw (5) is formed in the joint body, characterised in that the repositioning screw (5) engages on both sides of the joint body (14) through windows (16) in the wall of the connecting portion (2), that the joint body (14) is divided into two transversely to the axis (17) of the guide passage (15) and provided in one (14.1) of the two parts is the tapering seat (19) which is coaxial with the guide passage (15) and in which is disposed a clamping ring (20) which embraces the repositioning screw (5) and which is slit along a generatrix, and that in the clamping condition the clamping member braces the two joint body parts (14.1, 14.2) in the mounting opening (13) against the mounting surface (13') thereof and the clamping ring (20) in the tapering seat (19) against the repositioning screw (5).

2. An implant according to claim 1 characterised in that the tapering seat (19) opens with the enlarged end at the gap (18) between the two joint body parts (14.1, 14.2) and provided in the joint body part (14.2) which is opposite said mouth opening is a screwthreaded bore (21) which is coaxial with the guide passage (15) and in which is guided, as the clamping member, a screwthreaded sleeve (11) which forms a portion of the guide passage (15) and which bears with the sleeve end (22) that is within the joint body (14) against the clamping ring (20) disposed in the tapering seat (19) and at the other sleeve end projects outwardly through the window (16) and is accessible for actuation purposes.

3. An implant according to claim 1 or claim 2 characterised in that the clamping ring (20) has an external tapering surface (23) which is adapted to the tapering seat (19) and the edge (24) of the clamping ring, which is towards the screwthreaded sleeve (11), is of an outside diameter which is at least as large as the screwthreaded sleeve (11).

4. An implant according to one of claims 1 to 3 characterised in that the mounting opening (13) and the joint body (14) are of a cylindrical configuration with the cylinder axis (25) perpendicular to the axis (17) of the guide passage (15), that spring rings (27) which are slit along a generatrix are inserted into annular grooves (26) provided on both sides of the guide passage (15) at the outer peripheral surface of the joint body (14) coaxially with the cylinder axis (25), which spring rings are provided on the outside with transverse grooving (28) and project with the groove lands radially slightly beyond the cylindrical peripheral surface (29) of the joint body (14).

5. An implant according to claim 4 characterised in that the transverse grooving (28) is formed by a screwthread applied to the spring ring (27).

6. An implant according to claim 4 or claim 5 characterised in that when the connecting portion (2) is guided on a spindle (1) of the implant the cylinder axis (25) of the joint body (14) is perpendicular to the spindle axis.

## Revendications

1. Implant pour aligner et fixer réciproquement deux os ou parties d'os comme, par exemple, implant de spondylodèse pour la correction et la fixation de la position réciproque de vertèbres, comportant au moins deux éléments de raccordement (2) qui sont guidés l'un par rapport à l'autre et dont la distance réciproque est réglable dans la direction de guidage, et comportant des vis de repositionnement (5) pouvant être vissées dans les os ou les parties d'os, telles que des vertèbres, et pour lesquelles des logements (8) sont prévus dans les éléments de raccordement (2) pour la fixation de ces derniers sur les os ou parties d'os, telles que des vertèbres, et dans lequel les vis de repositionnement (5) sont guidées de manière à être déplaçables dans leur direction longitudinale dans leur logement respectif (8) et à pouvoir basculer transversalement par rapport à cette direction, et les logements (8) sont équipés d'un dispositif de serrage pouvant être actionné par un organe de serrage et qui, à l'état serré, bloque la vis de repositionnement (5) aussi bien contre une translation que contre un basculement dans le logement (8), et dans lequel un siège conique coaxial (19) est prévu et une ouverture de support (13) pour un corps d'articulation (14) est formé dans l'élément de raccordement (2) et un canal de guidage (15) pour la vis de repositionnement (5) est formé dans le corps d'articulation, caractérisé en ce que la vis de repositionnement (5) s'engage, des deux côtés du corps d'articulation (14), dans des fenêtres (16) prévues dans la paroi de l'élément de raccordement (2), que le corps d'articulation (14) est divisé en deux parties transversalement par rapport à l'axe (17) du canal de guidage (15) et que dans l'une (14.1) des deux parties est formé le siège conique (19), qui est coaxial au canal de guidage (15) et dans lequel est située une bague de serrage (20) qui entoure la vis de repositionnement (5) et est fendue longitudinalement le long d'une génératrice, et qu'à l'état serré, l'organe de serrage serre les deux parties (14.1, 14.2) du corps d'articulation dans l'ouverture de support (13), contre la surface d'appui (13') de cette ouverture et serre la bague de serrage (20) dans le siège conique (19) contre la vis de repositionnement (5).

2. Implant selon la revendication 1, caractérisé en ce que le siège conique (19) débouche par l'extrémité élargie dans la fente (18) présente entre les deux parties (14.1, 14.2) du corps d'articulation et que dans la partie (14.2) du corps d'articulation, qui est située à l'oppose de cette embouchure, est prévu un perçage taraudé (21) coaxial au canal de guidage (15) et dans lequel est guidée, en tant qu'organe de guidage, une douille filetée (11), qui forme une partie du canal de guidage (15) et qui vient en butée, par son extrémité (22) située à l'intérieur du corps d'articulation (14), contre la bague de serrage (20) située dans le siège conique (19) et qui fait saillie extérieurement, à son autre extrémité, à travers la fenêtre (16) et est accessible pour son actionnement.

3. Implant selon la revendication 1 ou 2, caractérisé en ce que la bague de serrage (20) possède une surface conique extérieure (23) adaptée au siège conique (19) et que le bord (24) de la bague de serrage, qui est tourné vers la douille filetée (11), possède un diamètre extérieur au moins égal à celui de la douille filetée (11).

4. Implant selon l'une des revendications 1 à 3, caractérisé en ce que l'ouverture de support (13) et le corps d'articulation (14) sont agencés avec une forme de cylindre dont l'axe (25) est perpendiculaire à l'axe (17) du canal de guidage (15) et que dans des gorges annulaires (26), qui sont aménagées des deux côtés du canal de guidage (15) dans la surface périphérique extérieure du corps d'articulation (14) coaxialement à l'axe (25) du cylindre, sont insérées des bagues élastiques (27) fendues le long d'une génératrice et qui sont pourvues, sur leur face extérieure, d'un rainurage transversal (28) et, par les barrettes présentes entre les rainures, font légèrement saillie radialement au-delà de la surface circonférentielle cylindrique (29) du corps d'articulation (14).

5. Implant selon la revendication 4, caractérisé en ce que le rainurage transversal (28) est formé par un filetage formé sur la bague élastique (27).

6. Implant selon la revendication 4 ou 5, caractérisé en ce que, lors du guidage de l'élément de raccordement (2) sur une broche (1) de l'implant, l'axe (25) du cylindre du corps d'articulation (14) est perpendiculaire à l'axe de la broche.
